# EUROPEAN PATENT SPECIFICATION

(11) **EP 0 673 267 B1**
(45) Date of publication and mention of the grant of the patent: **19.04.2000**
(21) Application number: 92924180.0
(22) Date of filing: 02.11.1992
(51) Int. Cl.: A61M 31/00

(54) **INFUSION PUMP, BAG AND METHOD OF USE**
INFUSIONSPUMPE, INFUSIONSBEUTEL UND ANWENDUNGSVERFAHREN
POMPE ET SACHET DE TRANSFUSION ET LEUR PROCEDE D'UTILISATION

(43) Date of publication of application: 27.09.1995
(73) Proprietor: MEDICATION DELIVERY DEVICES, San Diego, CA 92121 (US)
(72) Inventor: ROSS, Stephen, O., Vista, CA 92083 (US); MC WILLIAMS, Mark, D., San Diego, CA 92126 (US)
(74) Representative: Ruffles, Graham Keith
(86) International application number: US9209346
(87) International publication number: WO9409847

(56) References cited:
- US-A- 3 640 277
- US-A- 3 895 741
- US-A- 4 237 881
- US-A- 4 430 078
- US-A- 4 613 327
- US-A- 4 657 160
- US-A- 4 714 462
- US-A- 4 955 860
- US-A- 5 061 242

## Description

The present invention relates generally to apparatus and methods for the intravenous infusion of medical treatment fluid to a patient. More particularly, the present invention pertains to a pump for the intravenous infusion of medical treatment fluid to a patient, and methods for the use of such pump. In a particular aspect, the present invention relates to an intravenous infusion pump operated by inflation of a bladder which impinges against a flexible treatment fluid reservoir.

### BACKGROUND OF THE INVENTION

As illustrated by US Patents Nos 3,640,277; 3,895,741; 4,237,881; 4,430,078; and 4,657,160; intravenous administration sets which are useful for infusing medical treatment fluids to a patient are well known and widely used. In the most simple configuration, the intravenous fluid administration set includes a fluid source, an intravenous line connecting the fluid source to the patient, and a device operatively associated with the intravenous line to influence the rate of fluid flow to the patient. Flow rate control devices are characterized as either pumps or controllers. Controllers rely on gravity for the flow of treatment fluid through the system, while pumps exert an electro-mechanical force on the fluid to establish a fluid flow.

Pumps are often preferred because they can deliver higher pressures and can accurately deliver a wide range of flow rates. However, pumps have some inherent disadvantages which render them less than ideal, e.g., cost, complexity, size, and (lack of) mobility. There is, therefore, a need for pumps which are relatively small and simple to operate, yet which are relatively accurate and reliable (and which preferably utilize a minimum number of components in the fluid containment and delivery set thereof). The ability to produce such a pump which is portable and which is relatively inexpensive to manufacture would be beneficial in the field of medical treatment fluid delivery, especially in the home care setting.

### SUMMARY OF THE INVENTION

In one aspect, the present invention comprises an infusion pump for infusing a medical treatment fluid intravenously to a patient. In another aspect, the present invention comprises a method for infusing intravenous fluid to a patient. In yet another aspect, the present invention comprises novel treatment fluid bags useful with the invention pump.

The invention infusion pump comprises a collapsible treatment fluid bag and an inflatable drive fluid bladder juxtaposed therewith. The invention infusion pump further comprises a control assembly to controllably inflate the bladder, so as to establish (and maintain) a desired pressure in the treatment fluid bag. The treatment fluid bag and bladder are positioned between opposing containment members which cause the bladder to impinge against the bag as the bladder is inflated.

The treatment fluid bag has an outlet tube affixed thereto which enables treatment fluid to exit the bag as it is being collapsibly compressed by impingement of the bladder thereagainst. The outlet tube is in fluid communication with the patient to convey the treatment fluid to the patient where it is received intravenously. The effluent flow rate through said outlet tube can readily be determined once a set pressure is established in the fluid treatment bag, e.g., by using an outlet tube having a predetermined fixed cross-sectional area which is defined by the inside diameter of the tube, or alternatively by a fixed flow restriction positioned along the length of tubing.

The infusion pump of the present invention has numerous advantages over prior art pumps, combining many desirable features such as accuracy, low cost, low power requirements, ease of operation, and the like, into a light-weight, self-contained, relatively small unit. The invention unit can be readily reconfigured in a variety of ways to adapt to the requirements of different therapies. For example, a wide range of flow profiles, rates of fluid delivery, volumes of fluid delivered, and the like, can be accomodated by the invention infusion pump. The invention unit employs a treatment fluid bag which can be readily filled, with treatment fluid administered to the patient by way of a simple, reliable delivery system.

The invention pump and delivery system employing same have certain inherent safety features. For example, the pressure under which the treatment fluid is delivered to the patient is limited by the design of the pump, in contrast to prior art pumps which may expose the patient to the possibility of pressures as high as 35 psi or about 36 kg force cm⁻² (or higher) in the event of malfunction. In addition, the possibility of free flow of treatment fluid from the treatment fluid bag is substantially eliminated with the invention pump, whereas prior art pumps may subject the patient to the possibility of exposure to a free flow of treatment fluid in the event of malfunction.

The novel features of this invention, as well as the invention itself, both as to its structure and its operation, will be further understood from the accompanying drawings, taken in conjunction with the accompanying description.

### BRIEF DESCRIPTION OF THE DRAWINGS

Figure 1 is a perspective view of one embodiment of the infusion pump of the present invention.
Figure 2 is a cross-sectional view as seen along line 2-2 in Figure 1, wherein the cover plate of the infusion pump has been removed.
Figure 3 is a cross-sectional view of the cover plate for the invention infusion pump, as seen along line 3-3 in Figure 1.
Figure 4 is a schematic of the control assembly for the infusion pump shown in Figure 2.
Figure 5 is a broken cross-sectional view corresponding to the view of Figure 3 showing another embodiment of the infusion pump of the present invention.
Figure 6 is a schematic of the control assembly for the infusion pump shown in Figure 5.
Figure 7 is a perspective view of another embodiment of the infusion pump of the present invention.
Figure 8 is a schematic of a treatment fluid bag of the present invention.
Figure 9 illustrates two embodiments of multichambered fluid treatment bags contemplated by the present invention. Figure 9A is a sectional view illustrative of a stacked fluid container arrangement. Specifically shown are two independent fluid chambers positioned between the drive fluid bladder and the containment means.
Figure 9B is a top view of parallel fluid chambers in the fluid container. Specifically shown are three independent fluid chambers. These independent fluid chambers can be independently controlled to pass fluid by means of a manual clamp or occluder valve.
Figure 10 illustrates two embodiments of the activator means employed in conjunction with the fluid treatment bag of the present invention. Figure 10A illustrates an example construction employing a magnetically operated activator means wherein a Hall switch becomes actuated when the fluid treatment bag containing a soft iron bar therein is positioned within the containment means so as to complete the magnetic circuit.
Figure 10B illustrates an example of an optical activator means wherein light emitted from an LED is reflected onto a receiving detector by a surface associated with the fluid treatment bag, when the fluid treatment bag is properly positioned within the containment means of the invention pump. Thus, the presence of the reflective surface is indicative of the presence of the proper fluid treatment bag in the containment means.

### DETAILED DESCRIPTION OF THE INVENTION

In accordance with the present invention, there is provided a pump for infusing intravenous treatment fluid to a patient. The invention infusion pump comprises:
a collapsible treatment fluid bag;
an inflatable bladder juxtaposed with said treatment fluid bag;
means to vent said bladder;
means for impinging said bladder against said treatment fluid bag as said bladder is inflated;
an outlet tube in fluid communication with said treatment fluid bag and said patient; and
means for controlling the pressure of said bladder so as to collapse said treatment fluid bag and controllably expel treatment fluid therefrom.

In the pump of the present invention, the controlling means is for electronically controlling the pressure of said bladder and further comprises a co-ordinating activator means, so that positioning a fluid treatment bag containing an activator means in the containment for said bag allows said activator means to interact, thereby enabling the means for electronically controlling the pressure of said bladder to operate.

In accordance with another embodiment of the present invention, there is provided a collapsible fluid treatment bag comprising:
an attached extension set;
activator means capable of interacting with the means for electronically controlling the pressure of said bladder so as to enable the means for electronically controlling the pressure of said bladder to operate; wherein said means for electronically controlling the pressure of said bladder is disabled in the absence of said activator means; and
a port for introducing fluid into said treatment bag.

The collapsible treatment fluid bag contemplated for use in the practice of the present invention can be made of materials commonly employed to produce flexible bags of the type conventionally used to retain medical treatment fluids for intravenous infusion. Examples of such materials include polyvinyl chloride (PVC), polyolefins (e.g., polypropylene), polyurethanes, and the like. The treatment fluid bag is preferably filled before placement in the containment means which enables the inflated bladder to impinge the treatment fluid bag. Alternatively, treatment fluid bag can be placed in the containment means, then filled with treatment fluid. The treatment fluid bag can be filled to any suitable volumetric capacity; typically between about 100 ml and 5000 ml.

A variety of shapes are acceptable for the invention fluid treatment bag. Preferably, the bag will have a shape that facilitates the expulsion of air therefrom via the inlet or outlet tube. Shapes which serve this purpose are shapes which provide the bag with a smooth surface wherein substantially no pockets exist that would allow air to become trapped in the bag. Thus, fluid treatment bags having a tapered top surface, a domed top surface, a convex top surface, and the like, are contemplated.

Treatment fluid bags contemplated for use in the practice of the present invention can be viewed as modified versions of conventionally available treatment fluid bags. One such modification is the presence (on invention treatment fluid bags) of activator means capable of interacting with coordinating activator means, optionally included in the control assembly of the above-described infusion pump, so as to enable the control assembly of said pump to operate; wherein the control assembly (in this embodiment of the invention) is disabled in the absence of the activator means, i.e., the activator means is capable of serving as a "safety interlock", thereby preventing the inadvertent use of fluid containers not specifically designed to be used in conjunction with the invention pump. Such inadvertent use is undesirable as it could lead to excessively high flow rates for delivery of treatment fluid to the patient. Thus, the presence of the activator means ensures that the invention pump is operable for the delivery of treatment fluid to the patient only when a treatment fluid bag containing the desired treatment fluid is inserted into the containment means of the pump. Thus, only the desired treatment fluid is delivered to the patient, and such treatment fluid is provided at an appropriate target flow rate. At the same time, delivery of treatment fluids not contemplated for use with the particular configuration (i.e., flow rate, quantity of treatment fluid, etc) of the invention pump is prevented.

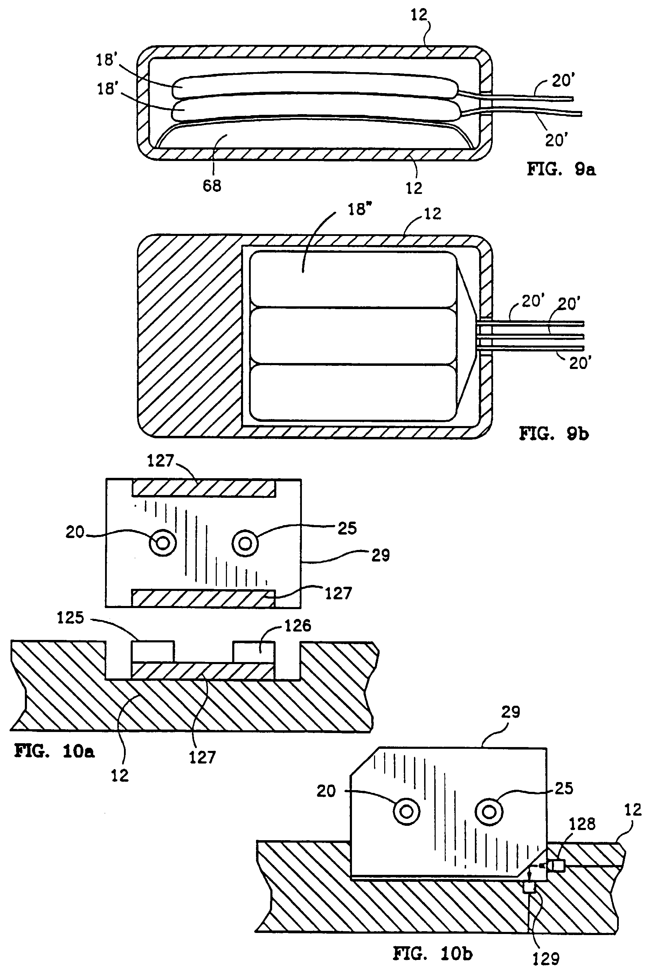

Another variation contemplated for fluid treatment bags of the invention is the use of multichambered bags. The multiple chambers can be associated in stacked or parallel orientation (see Figures 9A and 9B for examples of each orientation). Each of the individual chambers can be provided with their own independent ouput means, or a common output can be employed for the fluids contained in the individaul chambers. The delivery of fluid to a patient from the individual (or combined) contents of each chamber can be controlled in any suitable manner, e.g., by manual means, automatic means, or a combination thereof.

Examples of activator means useful in the practice of the present invention include mechanical means (e.g., lock and key operation, switch, bag configuration (so that only the appropriate bag size and configuration will fit in the containment means), and the like), magnetic means (e.g., positioning a magnet in the containment means and a piece of ferrite in the fluid treatment bag, so that positioning of the treatment fluid bag in the containment means enables the control circuit; see, for example, Figure 10A), electrical contact (so that appropriate positioning of the fluid treatment bag in the containment means completes a circuit required to enable the control circuit), optical coupling (so that the presence of the treatment fluid bag is required for the optical circuit to be completed; see, for example, Figure 10B), and the like.

In addition to the activator means, the extension set of the invention treatment fluid bag optionally further comprises a built-in flow restrictor; fitment for attaching the treatment fluid bag to a needle or to a patient's catheter site; clamp to stop the flow of the treatment fluid before, during or after delivery of the treatment fluid to the patient; a filter in the tubing line; a check valve; and the like.

Inflatable bladder(s) contemplated for use in the practice of the present invention are constructed so as to be readily expandable, i.e., having a shape which incurs substantially no strain energy as the bladder is expanded to a size which corresponds to substantially the entire volume of the containment means in which the treatment fluid bag and bladder are positioned. Exemplary materials from which bladders useful in the practice of the invention can be prepared include polymeric materials, rubber-filled or polymer-filled cloth, and the like. Accordion-type bladders, pre-folded or pre-creased bladders, or any of a variety of other collapsible configurations are suitable.

It is also desirable to be able to controllably vent the bladder, so as to reduce the flow rate of treatment fluid from the bladder, terminate flow of treatment fluid, allow disassembly and servicing of the invention pump, and the like. Such venting can be accomplished in a variety of ways. For example, a vent having a pinch clamp thereon can be provided on the bladder. Alternatively, the drive fluid pump can be a reversible pump, so that the pump can be employed to remove drive fluid from the bladder. As another alternative, the latch means used to secure the base to the cover of the invention pump assembly can serve multiple purposes, i.e., latching the pump assembly, activating the unit electronically, and closing off the vent means for the bladder. Optionally, there may also be provided (as part of the pump assembly) means to assist in deflating/flattening the bladder once inflation thereof has been discontinued and a vent opened. This can be accomplished in a variety of ways, such as, for example, by incorporating a light-duty spring within the bladder (to aid in drawing the bladder down to its non-inflated size), by including a light-duty spring in the containment means (to aid in returning the bladder to its non-inflated size), by providing means to evacuate the bladder, and the like.

The means for impinging the bladder against the treatment fluid bag as the bladder is inflated can be provided in a variety of ways. For example, such means can be provided by containment members, which may comprise a variety of structures. Optionally, one or more of the containment members will include a member complementary to the activator means of the treatment fluid bag, so that the juxtaposition of containment member(s) and treatment fluid bag will cooperate to enable the means for controllably inflating the bladder to operate (when the means for controllably inflating said bladder is disabled in the absence of said activator means).

In one embodiment, a first containment member is a rigid platen having a planar surface and a second containment member is a rigid base having a receptacle formed therein to retain the drive fluid bladder and to receive the treatment fluid bag. The base and platen are removably engageable to fully enclose the bag and bladder. Guide members are preferably provided on the platen and base to enable slidable engagement thereof after placement of fluid treatment bag in the base. Preferably, a latch is further provided to securely lock engagement of the platen and base in place.

In another embodiment, a first containment member is a rigid platen having a receptacle formed therein to retain the drive fluid bladder and to receive the treatment fluid bag, and a second containment member is a rigid base having a planar surface. As with the previously described embodiment, the base and platen are removably engageable to fully enclose the bag and bladder. Guide members are preferably provided on the platen and base to enable slidable engagement thereof after placement of a fluid treatment bag in the platen. Preferably, a latch is further provided to securely lock engagement of the platen and base in place.

In yet another embodiment, a first containment member is a rigid platen having a planar surface. A second containment member is a flexible, yet substantially inelastic sheet, loosely attached to the platen at opposite sides to form a sling. The bladder is positioned in the sling and the infusion pump is assembled by sliding a treatment fluid bag into the sling in abutment with the bladder, and optionally attaching the outlet tube to the bag. In a preferred embodiment, the treatment fluid bag employed will include its own pre-attached extension set, optionally containing its own flow restrictor.

Containment members contemplated for use in the practice of the present invention can be formed from high-strength materials such as rigid plastics, flexible plastics, semi-rigid plastics, die-cast aluminum, and the like.

Means for controllably inflating the drive fluid bladder contemplated for use in the practice of the present invention are provided by a control assembly, which comprises a pressure sensor, in operative communication with a suitable control circuit, which is, in turn, in operative communication with a drive fluid pump. Optionally, the control assembly may contain a display for operator interface. Optionally, control assembly may also include a coordinating activator means, i.e., a member complementary to the activator means of the treatment fluid bag, so that the juxtaposition of the control assembly and treatment fluid bag will cooperate to enable the control assembly to operate (when the control assembly is disabled in the absence of such activator means associated with the fluid treatment bag).

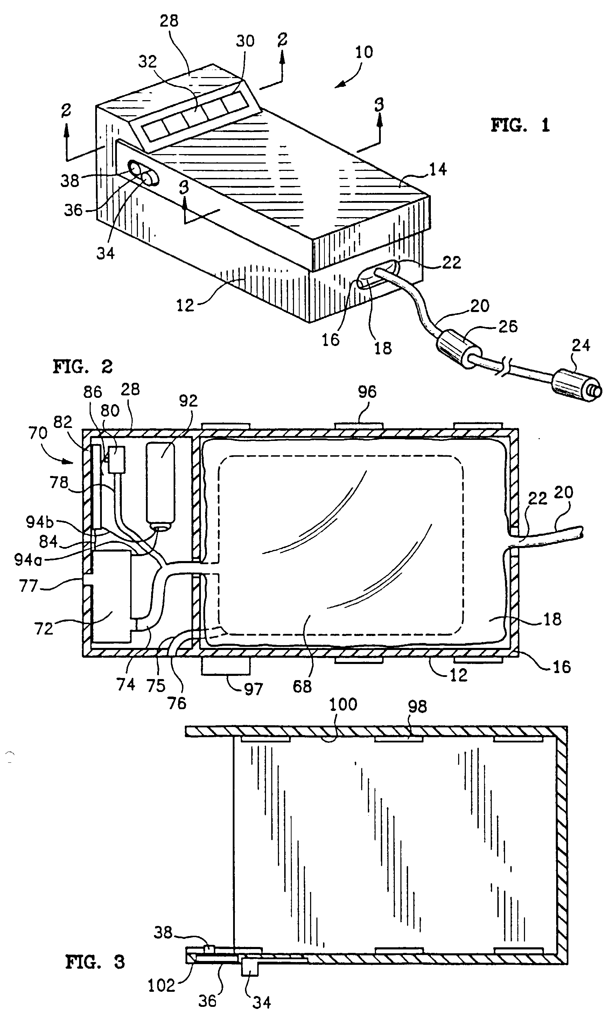

The control assembly can be provided as an integral part of the pump of the invention, or can be provided as a separate module, so long as communication means is provided to introduce drive fluid from the drive fluid pump into the drive fluid bladder. Housing can be provided for the control assembly to be positioned internal to such housing, as shown, for example, in Figure 1. Such control assembly housing can further be integral with the base and/or cover of the pump assembly. The pump assembly can further comprise an input station comprising a plurality of touch keys for entering operational commands to the control assembly. Alternatively, separate housing can be provided for the control assembly which is then employed as a separate module positioned in convenient proximity to the containment means in which the treatment fluid bag and drive fluid bladder are positioned.

Control circuits contemplated for use in the practice of the present invention provide means to establish a set point pressure, means for comparing the set point pressure to the actual pressure in the bladder (e.g., a comparator), and means capable of driving the drive fluid pump motor (e.g., a switch to turn on the motor). Such a control circuit can optionally contain an op-amp to amplify the pressure signal from the pressure sensor. Alternatively, a pressure switch can be employed to control the operation of the drive fluid pump.

In addition, control circuits contemplated for use in the practice of the present invention optionally further include means for monitoring the amount of fluid dispensed over time. This can be accomplished in a variety of ways, such as, for example, by monitoring the amount of time the pump is on during the period of delivery, by monitoring the number of revolutions the pump has made during the period of delivery, by monitoring the duty cycle of the pump during the period of delivery, and the like. Each of the above-described measures are proportional to the volume displaced by the pump, and thus can be used to readily determine the actual volume of fluid displaced during the time period of interest.

The additional information provided by monitoring the total volume of fluid delivered by the pump, in addition to controlling the rate of fluid delivery, allows one to check the operation of the pump over an extended period of time. Thus, after the pump has been in operation for a period of time, the actual volume of fluid delivered can be compared to the volume which the pump has theoretically delivered (based on the set delivery rate for the pump). If the desired volume of fluid has not been delivered, the flow rate can be adjusted accordingly. Alternatively, if a target total volume of fluid is sought to be delivered in a defined period of time, the ability to monitor the actual volume delivered over time would be useful to ensure that the desired volume of treatment fluid is actually delivered within the desired timeframe.

Drive fluid pumps contemplated for use in the practice of the present invention are readily available and are well known in the art of automatic blood pressure monitors. Any drive means capable of producing a positive displacement of drive fluid on each stroke can be employed. Examples include an eccentric cam driving a diaphragm, a bellows, and the like; various solenoid configurations driving a diaphragm, a bellows, and the like; a stepper motor with an axial lead screw shaft driving a diaphragm, a bellows, and the like; a rotary vein-type pump driven by a DC motor, a stepper motor, and the like; and so on.

Conventional flexible plastic medical tubing having a predetermined fixed inner diameter is suitable for use as the outlet tube employed in the practice of the present invention. A flow restrictor may optionally be positioned along the length of such tubing, intervening in the treatment fluid flowpath of the outlet tube. The presence of a flow restrictor serves to further narrow the inner diameter of the outlet tube to a reduced fixed inner diameter.

The treatment fluid flow rate through the outlet tube can be controlled in a variety of ways. According to one such control method, the treatment fluid flow rate is adjusted either up or down by correspondingly increasing or decreasing the drive fluid pump output and consequently the pressure in the bladder. For this purpose a pressure sensor is provided in fluid communication with the drive fluid bladder. The pressure sensor supplies pressure measurements to a control circuit regulating the pump output. Both the pressure sensor and control circuit are preferably retained within a control housing integral with one or both containment members. A command input station may also be provided on the housing in communication with the control circuit to enable an operator to program a specific sequence of operating parameters into the control circuit.

According to an alternate control method, the treatment fluid flow rate can be adjusted by increasing or decreasing the opening of the outlet tube by means of an occluder or restrictor positioned therealong. For example, the fluid flow rate can be adjusted to one of two levels, i.e., either full flow or no flow, by correspondingly opening or closing the outlet tube by means of an occluder. The occluder is preferably a clamp external to the outlet tube which operates by pinching off the flexible outlet tube to close it and releasing the tube to open it. For this purpose a control circuit is provided to dictate the position of the occluder. An input station in communication with the control circuit may also be provided to enable the operator to program a specific sequence of occluder operation as a function of time.

As those of skill in the art recognize, combination(s) of the above-described pressure control/flow control methodology can also be employed. For example, a flow profile defined by the drive fluid pump output can be further modified by the operation of an occluder or restrictor in combination therewith.

Optionally, the invention infusion pump further comprises a bubble detector, so as to ensure that substantially air-free fluid is delivered to the patient. Numerous means exist to detect the presence of bubbles in fluid-bearing tubing (e.g., infrared detection, ultrasonic detection, and the like), and those of skill in the art can readily select a suitable means for use with the invention infusion pump.

By employing a combination of control means in the practice of the present invention, external inputs from a variety of sources (e.g., manual input by the patient or a care-giver, electronic input derived from a suitable sensor (e.g., to monitor heartrate, blood pressure, blood chemistry, etc.), and the like) can be employed to adjust the flow rate at which treatment fluid is delivered to the patient.

Referring now to Figure 1, a first embodiment of an infusion pump of the present invention is shown and generally designated **10**. Infusion pump **10** is housed within a pair of containment members, the lower containment member **12** being the base and the upper containment member **14** being a cover plate termed a platen. Retained within receptacle **16** formed by base **12** is a prefilled collapsible treatment fluid bag **18**. Extending from bag **18** is a flexible outlet tube **20** which exits receptacle **16** via opening **22** in base **12**. Tube **20** extends to a patient in whom the treatment fluid is to be intravenously infused. A site adaptor fitting **24** is provided at the end of tube **20** which enables communication between tube **20** and a vein of the patient.

Tube **20** is a conventional flexible plastic medical tubing having a predetermined fixed inner diameter. A flow restrictor **26** may optionally be positioned along tube **20** which intervenes in the treatment fluid flowpath of tube **20** to further narrow the inner diameter of tube **20** as originally manufactured to a reduced fixed inner diameter.

As shown in Figure 1, housing **28** can be provided for a control assembly which, in this embodiment, is positioned internal to housing **28**, as described hereafter. Control assembly housing **28**, in this embodiment, is integral with base **12** and has an input station **30** on its face comprising a plurality of touch keys **32** for entering operational commands to the control assembly.

Positioned on cover plate **14** is a latch **34** extending from a latch opening **36** in plate **14**. Latch **34** is provided for locking cover plate **14** in place upon engagement with base **12**. Drive fluid vent **38** (formed in base **12**) is preferably positioned in alignment with latch opening **36** and latch **34** when cover plate **14** engages base **12**. Vent **38** enables discharge of drive fluid from a drive fluid bladder, which is retained within receptacle **16** described hereafter with reference to Figure 2, when latch **34** is in the unlocked position as shown. Once latch **34** is slid into the locked position over vent **38**, discharge of drive fluid is blocked.

In accordance with this embodiment, infusion pump **10** is further described with reference to Figure 2 which shows the pump components internal to control assembly housing **28** and base **12** having cover plate **14** removed therefrom. Collapsible fluid treatment bag **18** is positioned to easily fit within receptacle **16** with outlet tube **20** extending therefrom via opening **22**. Positioned beneath bag **18** and juxtaposed therewith is inflatable bladder **68** (shown in a deflated state). Operation of bladder **68** is under the control of a control assembly generally designated **70** which is contained within housing **28**.

Assembly **70** comprises a drive fluid pump **72** in fluid communication with bladder **68** via drive fluid inlet tube **74**. Bladder **68** has a drive fluid outlet tube **75** in fluid communication with a drive fluid outlet port **76**. Pump **72** receives drive fluid from an external source, such as the ambient atmosphere, via drive fluid feed port **77**. A pressure sensor tube **78** is in fluid communication with drive fluid inlet tube **74** to provide fluid communication between pressure sensor **80** and bladder **68**. Pressure sensor **80** may be a pressure transducer having a linear output or a pressure switch having an on/off nonlinear output. Sensors suitable for this purpose include solid state sensors, mechanical sensors, capacitive sensors, reluctance-type sensors, and the like. Operational commands for assembly **70** are generated in a control circuit, e.g., a microprocessor, integral with electronics board **82**. The control circuit may be preprogrammed or alternatively reprogrammable by means of input station **30** shown in Figure 1.

Electronics board **82** electrically communicates with pump **72** and sensor **80** via lines **84** and **86**, respectively. The components of assembly **70** can all be powered from a single electrical power source **92** which distributes power via lines **94a**, **94b**. Power source **92** may be an internal battery as shown here which is either replaceable, rechargeable, or simply disposable along with the entire infusion pump **10** upon use. Alternatively, the power source can be external to assembly **70** such as common household current.

Base **12** and cover plate **14** can be fixably engaged in a variety of ways, such as, for example, by a combination of pivot means and latching means (wherein pivot means and latching means can be positioned on the front, side, or back of the base and/or cover plate, wherein the pivot means and latching means are preferably positioned on opposing sides of the base and/or cover plate), by slidable engagement of base and cover plate in combination with pivot means, by use of a latch which is actuated by the bladder as the bladder is inflated, and the like. As shown in Figure 2 (which shows cover plate **14**), a presently preferred means of such attachment comprises providing rail segments **96** and latch coupling **97** on the outside edge of base **12**. The rail segments and latch coupling enable fixable engagement of base **12** and cover plate **14**. Rail segments **98** are provided along the bottom of inside edge **100** of cover plate **14**. Rail segments **98** are positioned to align beneath rail segments **96** of base **12** when cover **14** is slid over receptacle **16** of base **12**. Latch **34** is positioned within latch opening **36** to lock rail segments **96, 98** in place relative to each other when latch **34** is hooked through slot **102** and over latch coupling **97** on base **12**. When latch **34** is in the locked position, it plugs drive fluid vent **38** of cover **14** which is in alignment with port **76** of base **12** in fluid communication with bladder **68**.

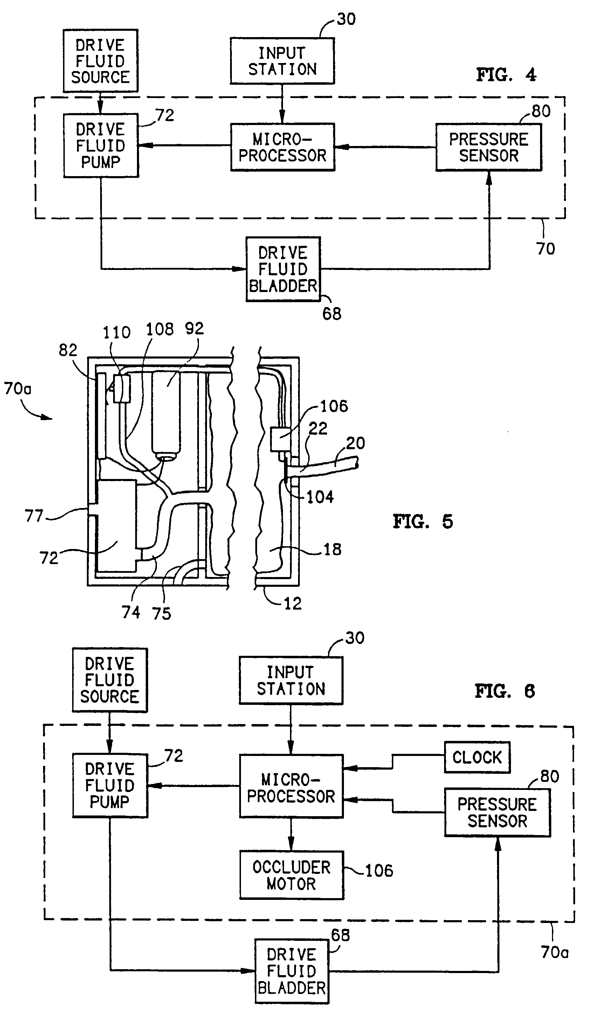

Figure 5 shows an alternate embodiment of a control assembly generally designated **70a** wherein occluder **104** is provided on tube **20** immediately inside opening **22** to enable closure of tube **20** and stoppage of treatment fluid flow therethrough as desired. An occluder motor **106** (e.g., a solenoid, a latching solenoid, a step-motor actuator, various mechanisms in cooperation with a motor, such as an over-the-center mechanism, eccentric cam actuator, and the like) adjacent occluder **104** and in communication with power source **92** and suitable control circuit, e.g., microprocessor, via lines **108** and **110,** respectively, activates occluder **104** on command from the control circuit. A clock is further provided integral with electronics board **82** to provide control input to the control circuit. It is to be understood that although control assemblies **70** and **70a** have been described above with reference to infusion pump **10**, similar control assemblies can be provided with infusion pump **40** to perform the same function.

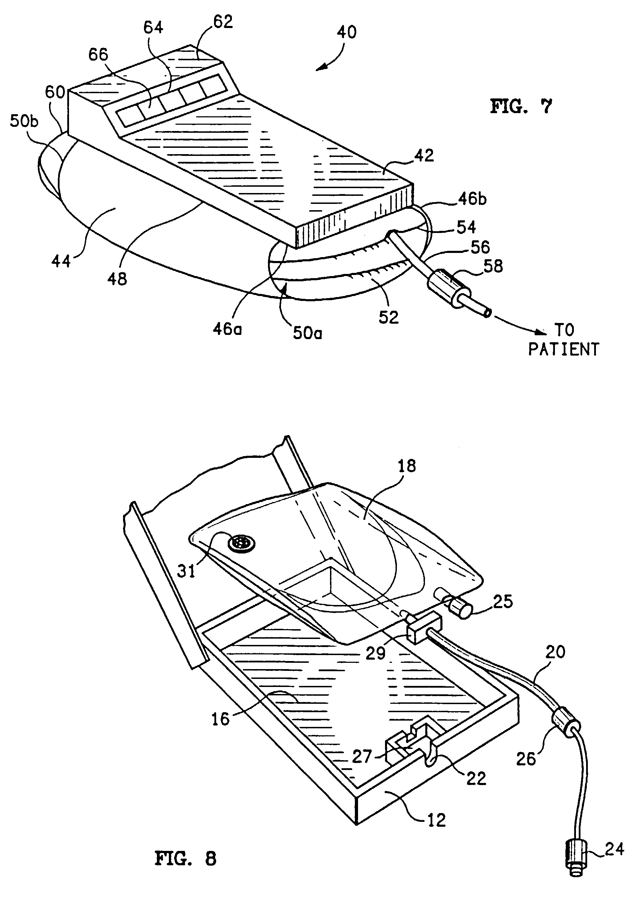

Figure 7 shows an alternate embodiment of the infusion pump of the present invention generally designated **40**. Infusion pump **40**, like infusion pump **10**, has a pair of containment members including a rigid platen **42** as the upper containment member. The lower containment member **44**, however, is a flexible, yet substantially inelastic and unstretchable sling preferably formed from a high-strength mesh or cloth fabric, such as a nylon mesh. Sling **44** has a rectangular shape having two opposing sides **46a, 46b** fastened to the bottom **48** of platen **42** such that sling **44** hangs loosely therefrom with openings **50a, 50b** at the front and back of sling **44**, respectively.

An inflatable drive fluid bladder **52** is retained in sling **44**. A collapsible treatment fluid bag **54** is removeably positioned in sling **44** juxtaposed with bladder **52**. Positioning of bag **54** in sling **44** is accomplished by sliding prefilled bag **54** into sling **44** through either opening **50a** or **50b**, while bladder **52** is retained therein. Extending from bag **54** to the patient is treatment fluid outlet tube **56** having a site adaptor fitting (not shown) positioned at the end of tube **56** opposite bag **54** which enables intravenous infusion of the treatment fluid into the patient.

Tube **56** is a conventional flexible plastic medical tubing having a predetermined fixed inner diameter. A flow restrictor **58** may optionally be positioned along tube **56**. Platen **42** is formed from a high-strength rigid material such as a rigid plastic. Collapsible bag **54** is a flexible plastic bag of the type conventionally used to retain medical treatment fluids for intravenous infusion. Bag **54** typically has a volumetric capacity larger than that of bag **18** of the first embodiment and is preferably between about 1000 ml and 5000 ml.

Inflatable drive fluid bladder **52** has a drive fluid tube **60** extending therefrom, through opening **50b**, and into control assembly housing **62** where tube **60** connects to a drive fluid pump, thereby providing fluid communication between bladder **52** and the drive fluid pump described with reference to Figure 2. Control assembly housing **62** is integral with platen **42** and has an input station **64** on its face comprising a plurality of touch keys **66** for entering operational commands to the control assembly contained within housing **62**.

As used herein, the term "portable" pumps refers to pumps which are small enough (and lightweight enough) to carry around without the need for special transport means, operate on their own power source (without the need for an independent source of power), are rugged enough to withstand the impact of constant and/or frequent movement, and the like.

An exemplary treatment fluid bag of the invention is shown in Figure 8 wherein **12** refers to the base in which the treatment fluid bag is retained, **16** refers to the receptacle formed by base **12, 18** refers to the treatment fluid bag, **20** refers to flexible outlet tube, **22** refers to the opening in base **12** wherethrough the flexible outlet tube passes, **26** refers to the optional flow restrictor, **24** refers to a site adaptor fitting, **25** refers to the fill port; **31** refers to an air vent; and **27** and **29** refer to means to incorporate activator means into the containment means and treatment fluid bag of the invention, respectively.

### OPERATION Operation of pump 10 is now described with reference

to Figures 1-5, it being further understood that the present operational description applies similarly to pump **40**, as well as to other variations. Infusion of treatment fluid is performed in one instance by placing a prefilled treatment fluid bag **18** in receptacle **16** atop bladder **68** after cover **14** is removed from base **12**. Outlet tube **20** is fed through opening **22**.

Cover **14** is slid back onto base **12** such that rail segments **96** engage rail segments **98**. The tight fit of bag **18** in receptacle **16** impinges bag **18** against bladder **68** as cover **14** is being positioned driving any residual drive fluid retained in bladder **68** out into the surrounding atmosphere via tube **74**, port **76**, and vent **38**. The preferred drive fluid is ambient air. Latch **34** is then slid shut to lock cover **14** in place and seal off vent **38** from the atmosphere. Closing of latch **34** can cooperate with a switch (not shown) to activate drive fluid pump **72**. In an alternate embodiment drive fluid pump **72** can be activated by the operator through a command to input station **30** which communicates with the control circuit of electronics board **82**.

When drive fluid pump **72** is activated, it draws air through drive fluid feed port **77** and feeds it under pressure to bladder **68** via tube **74**. The pressurized air inflates bladder **68** within the confined receptacle **16** impinging both bladder **68** and bag **18** against the inner surfaces of base **12** and cover **14**. Once the treatment fluid bag is pressurized, the contents thereof can be purged of air (e.g., by opening the clamp and letting air out of the bag, then closing the clamp) and placed in communication with a desired vein of a patient using site adaptor **24** at the end of tube **22**.

Bag **18** collapses at a rate proportional to the rate at which treatment fluid is expelled from the bag, thereby giving bladder **68** increasing amounts of space in which to expand. Bladder **68** can be inflated until it fills the entire volume of confined receptacle **16**, thereby completely collapsing bag **18** and expelling treatment fluid therefrom, thereby delivering treatment fluid to the patient.

In the embodiment of Figure 2, the flow rate of treatment fluid to the patient is governed by the fixed cross-sectional area of outlet tube **20** and the pumping pressure of drive fluid pump **72**. A cross-sectional area of the tube can be selected other than its area of manufacture by fixing flow restrictor **58** on tube **20** to reduce its cross-sectional area. Variable flow rate adjustment is achieved by means of control assembly **70** shown in Figure 2 and further shown schematically in Figure 4 which acts as a feedback circuit. Whenever pressure sensor **80** measures too low a pressure, it is detected by the control circuit which sends a signal to drive fluid pump **72** to increase its output up to the desired level. If the measured pressure is too high, the control circuit conversely sends a signal to drive fluid pump **72** to decrease its output accordingly.

In the embodiment of Figure 5 (also shown schematically in Figure 6), treatment fluid flow rate is regulated by fully opening or closing tube **20** in correspondence with occluder **104**. Operation of occluder **104** is governed by commands from the control circuit integral with electronics board **82** which further utilizes the clock also integral with electronics board **82** to determine tube **20** opening and closing times. In either control assembly embodiment, the operator can program additional control commands into the control circuit via input station **30** if desired.

The materials for construction of pumps **10** and **40** are generally and preferably selected to be both inexpensive and lightweight. This enables the pump of the present invention to be portable and, if desired, disposable after each of one or several uses.

While the particular infusion pumps as herein shown and disclosed in detail are fully capable of obtaining the objects and providing the advantages herein before stated, the disclosure is to be understood as merely illustrative of the presently preferred embodiments of the invention.

## Claims

1. A pump (10 or 40) for infusing intravascular treatment fluid to a patient, said infusion pump (10 or 40) comprising:
a collapsible treatment fluid bag (18);
an inflatable bladder (68) juxtaposed with said treatment fluid bag (18);
means (38) for venting said bladder (68);
means (12, 14 or 42, 44) for impinging said bladder (68) against said treatment fluid bag (18) as said bladder (68) is inflated;
an outlet tube (20) in fluid communication with said treatment fluid bag (18) and said patient; and
means (70 or 70a) for controlling the pressure of said bladder (68) so as to collapse said treatment fluid bag (18) and controllably expel treatment fluid therefrom;
characterized in that said controlling means (70 or 70a) is for electronically controlling the pressure of said bladder (68) and further comprises a co-ordinating activator means, so that positioning a fluid treatment bag (18) containing an activator means in the containment for said bag (18) allows said activator means to interact, thereby enabling the means (70 or 70a) for electronically controlling the pressure of said bladder (68) to operate.

2. A pump (10 or 40) according to claim 1 wherein said outlet tube (20) has a predetermined and substantially fixed cross-sectional area.

3. A pump (10 or 40) according to claim 1 further comprising an occluder (104) for selectively blocking treatment fluid flow across said outlet tube (20).

4. A pump (10 or 40) according to claim 1 wherein said means (70 or 70a) for electronically controlling the pressure of said bladder (68) comprises a pressure sensor (80), in operative communication with a suitable control circuit, which is, in turn, in operative communication with a drive fluid pump (72).

5. A pump (10 or 40) according to claim 4 further comprising an internal power source (92) for said means (70 or 70a) for electronically controlling pressure of said bladder (68).

6. A pump (10 or 40) according to claim 1 wherein said means (12, 14 or 42, 44) for impinging comprises first and second opposing containment members confining said juxtaposed bladder (68) and fluid bag (18).

7. A pump (10 or 40) according to claim 6 wherein said first containment member (42) has a rigid planar surface and said second containment member (44) is a flexible sheet engaging said first containment member.

8. A pump (10 or 40) according to claim 6 wherein said first containment member (12) is a rigid base and said second containment member is a rigid cover plate (14) removably engageable with said base to enclose said bag (18) and bladder (68) therein.

9. A pump (10 or 40) according to claim 1 further comprising means for monitoring the amount of fluid dispensed over time.

10. A collapsible fluid treatment bag (18) comprising:
an attached extension set;
activator means capable of interacting with the means (70 or 70a) for electronically controlling the pressure of the bladder (68) of a pump (10 or 40) according to claim 1, wherein said means (70 or 70a) for electronically controlling the pressure of said bladder (68) is disabled in the absence of said activator means; and
a port for introducing fluid into said treatment bag (18).

11. A treatment bag (18) according to claim 10 wherein said extension set comprises a built-in flow restrictor (26 or 58); fitment (24) for attaching a needle or a patient's catheter site; clamp to stop the flow of the treatment fluid before, during or after delivery of the treatment fluid to the patient; and, optionally, a particle eliminating filter in the tubing line and optionally an air venting filter mounted on treatment fluid bag (18) or in the tubing line, and optionally a check valve.

12. A treatment bag (18) according to claim 11 wherein said built-in flow restrictor is a fixed flow restrictor or a variable flow restrictor with preset flow rates.

13. A treatment bag (18) according to claim 10 wherein said activator means electronically, mechanically, pneumatically, magnetically, or optically interacts with the control mechanism of said pump (10 or 40).

14. A treatment fluid bag (18) according to claim 10 wherein said bag (18) has multiple chambers capable (18' or 18'') of holding treatment fluid therein.

15. A treatment fluid bag (18) according to claim 10 wherein said bag (18) has a shape that facilitates the expulsion of air therefrom via the inlet (25) or outlet(20) tube.

16. A treatment fluid bag (18) according to claim 14 wherein the multiple chambers (18') of the bag (18) are disposed in a stacked fluid container arrangement.

17. A treatment fluid bag (18) according to claim 14 wherein the multiple chambers (18'') of the bag (18) are disposed in a parallel orientation to one another.

18. A pump (10 or 40) for infusing intravascular treatment fluid to a patient, said infusion pump (10 or 40) comprising:
(1) a containment receptacle for receiving a removable, collapsible treatment fluid bag (18) having an outlet tube (20) for establishing fluid communication between said treatment fluid bag (18) and said patient; and
(2) an inflatable bladder (68) juxtaposed with said treatment fluid bag (18) when said treatment fluid bag (18) is positioned within said receptacle, such that said bladder (68) apples pressure to said treatment fluid bag (18) as said bladder (68) is inflated;
characterized by
(3) an electronic control system (70 or 70a) for electronically controlling the pressure of said bladder (68) so as to collapse said treatment fluid bag (18) and controllably expel treatment fluid therefrom through said outlet tube (20), wherein said means (70 or 70a) for electronically controlling the pressure of said bladder (68) and further comprises a co-ordinating activator means, so that positioning a fluid treatment bag (18) containing an activator means in the containment for said bag (18) allows said activator means to interact, thereby enabling the means (70 or 70a) for electronically controlling the pressure of said bladder (68) to operate.

19. A pump (10 or 40) according to claim 18, wherein a compatible treatment fluid bag (18) includes an activator, and said electronic control system further (70 or 70a) concludes interaction means for interacting with said activator when present on a treatment fluid bag (18) positioned within said receptacle to enable said electronic control system, said electronic control system being disabled in the absence of said activator.

20. A pump (10 or 40) according to claim 18, wherein said electronic control system (70 or 70a) includes a pressure sensor (80), in operative communication with a suitable electronic control circuit, which is, in turn, in operative communication with a drive fluid pump (72) operatively coupled to said bladder (68).

21. A pump (10 or 40) according to claim 18, further including means (38) for venting said bladder (68).

## Patentansprüche

1. Pumpe (10 oder 40) zur Infusion eines Intravaskulärbehandlungsfluids bei einem Patienten, wobei die Infusionspumpe (10 oder 40) folgendes aufweist:
einen faltbaren Behandlungsfluid-Beutel (18);
eine aufblasbare Blase (68) in Juxtaposition mit dem Behandlungsfluid-Beutel (18);
Mittel (38) zum Entlüften der Blase (68);
Mittel (12, 14 oder 42, 44) für das Anprallen der Blase (68) an den Behandlungsfluid-Beutel (18), wenn die Blase (68) aufgeblasen wird;
einen Auslaßschlauch (20) in Fluidverbindung mit dem Behandlungsfluid-Beutel (18) und dem Patienten; und
Mittel (70 oder 70a) zur Steuerung des Drucks der Blase (68), um so den Behandlungsfluid-Beutel (68) zusammenzufalten und das Behandlungsfluid kontrollierbar aus diesem auszustoßen;
dadurch gekennzeichnet, daß die Steuermittel (70 oder 70a) Mittel zur elektronischen Steuerung des Drucks der Blase (68) sind und außerdem koordinierende Aktivatormittel aufweisen, so daß durch das Einsetzen eines Behandlungsfluid-Beutels (18), in dem sich Aktivatormittel befinden, in das Gehäuse für den Beutel (18) die Aktivatormittel in Wechselwirkung treten können, wodurch die Mittel (70 oder 70a) zur elektronischen Steuerung des Drucks der Blase (68) zu arbeiten in die Lage versetzt werden.

2. Pumpe (10 oder 40) nach Anspruch 1, worin der Auslaßschlauch (20) eine vorher festgelegte und im wesentlichen feststehende Querschnittsfläche hat.

3. Pumpe (10 oder 40) nach Anspruch 1, die außerdem ein Verschlußelement (104) zum selektiven Blockieren des Flusses des Behandlungsfluids durch den Auslaßschlauch (20) aufweist.

4. Pumpe (10 oder 40) nach Anspruch 1, worin die Mittel (70 oder 70a) zur elektronischen Steuerung des Drucks der Blase (68) einen Drucksensor (80) in funktioneller Verbindung mit einer geeigneten Steuerschaltung aufweisen, die wiederum in funktioneller Verbindung mit einer Antriebsfluid-Pumpe (72) steht.

5. Pumpe (10 oder 40) nach Anspruch 4, die außerdem eine innen befindliche Energiequelle (92) für die Mittel (70 oder 70a) zur elektronischen Steuerung des Drucks der Blase (68) aufweist.

6. Pumpe (10 oder 40) nach Anspruch 1, worin die Mittel (12, 14 oder 42, 44) für das Anprallen erste und zweite gegenüberliegende Gehäuseelemente umfassen, welche die in Juxtaposition angeordnete Blase (68) und Fluidbeutel (18) umschließen.

7. Pumpe (10 oder 40) nach Anspruch 6, worin das erste Gehäuseelement (42) eine starre, plane Oberfläche hat und das zweite Gehäuseelement (44) ein flexibles Blech ist, das mit dem ersten Gehäuseelement ineinandergreift.

8. Pumpe (10 oder 40) nach Anspruch 6, worin das erste Gehäuseelement (12) eine starre Basis ist und das zweite Gehäuseelement eine starre Abdeckplatte (14) ist, die lösbar mit der Basis ineinandergreifen kann, um den Beutel (18) und die Blase (68) darin zu umschließen.

9. Pumpe (10 oder 40) nach Anspruch 1, die außerdem Mittel zur Überwachung der Menge des über die Zeit abgegebenen Fluids aufweist.

10. Faltbarer Behandlungsfluid-Beutel (18), der folgendes aufweist:
ein angebautes Erweiterungsset;
Aktivatormittel, die dafür geeignet sind, mit den Mitteln (70 oder 70a) zur elektronischen Steuerung des Drucks der Blase (68) einer Pumpe (10 oder 40) nach Anspruch 1 in Wechselwirkung zu treten, worin die Mittel (70 oder 70a) zur elektronischen Steuerung des Drucks der Blase (68) beim Fehlen der Aktivatormittel außer Betrieb gesetzt sind; und
eine Öffnung zur Einführung von Fluid in den Behandlungsbeutel (18).

11. Behandlungsbeutel (18) nach Anspruch 10, worin das Erweiterungsset eine eingebaute Durchflußdrossel (25 oder 58); eine Fassung (24) zur Befestigung einer Nadel oder der Patientenanschlußseite eines Katheters; eine Klemme zum Stoppen des Flusses des Behandlungsfluids vor, während oder nach der Abgabe des Behandlungsfluids an den Patienten; und, wahlweise, einen Partikel ausschaltenden Filter in der Schlauchleitung und, wahlweise, einen Entlüftungsfilter, der an dem Behandlungsfluid-Beutel (18) oder in der Schlauchleitung angebracht ist, und, wahlweise, ein Rückschlagventil aufweist.

12. Behandlungsbeutel (18) nach Anspruch 11, worin die eingebaute Durchflußdrossel eine nicht verstellbare Durchflußdrossel oder eine variable Durchflußdrossel mit voreingestellten Durchflußgeschwindigkeiten ist.

13. Behandlungsbeutel (18) nach Anspruch 10, worin die Aktivatormittel elektronisch, mechanisch, pneumatisch, magnetisch oder optisch mit dem Steuermechanismus der Pumpe (10 oder 40) in Wechselwirkung treten.

14. Behandlungsfluid-Beutel (18) nach Anspruch 10, worin der Beutel (18) mehrere Kammern (18' oder 18'') hat, die in der Lage sind, darin das Behandlungsfluid aufzunehmen.

15. Behandlungsfluid-Beutel (18) nach Anspruch 10, worin der Beutel (18) eine Form hat, die das Ausstoßen von Luft aus diesem über den Einlaß- (25) oder Auslaßschlauch (20) erleichtert.

16. Behandlungsfluid-Beutel (18) nach Anspruch 14, worin die mehreren Kammern (18') des Beutels (18) in einer gestapelten Fluidbehälter-Anordnung angeordnet sind.

17. Behandlungsfluid-Beutel (18) nach Anspruch 14, worin die mehreren Kammern (18'') des Beutels (18) in einer parallelen Ausrichtung zueinander angeordnet sind.

18. Pumpe (10 oder 40) zur Infusion eines Intravaskulärbehandlungsfluids bei einem Patienten, wobei die Infusionspumpe (10 oder 40) folgendes umfaßt:
(1) ein Gehäusebehältnis zur Aufnahme eines herausnehmbaren, faltbaren Fluidbeutels (18), der einen Auslaßschlauch (20) zur Herstellung der Fluidverbindung zwischen dem Behandlungsfluid-Beutel (18) und dem Patienten hat; und
(2) eine aufblasbare Blase (68) in Juxtaposition mit dem Behandlungsfluid-Beutel (18), wenn der Behandlungsfluid-Beutel (18) in das Behältnis eingesetzt ist, derartig, daß die Blase (68) Druck auf den Behandlungsfluid-Beutel (18) ausübt, wenn die Blase (68) aufgeblasen wird;
gekennzeichnet durch:
(3) ein elektronisches Steuersystem (70 oder 70a) zur elektronischen Steuerung des Drucks der Blase (68), um so den Behandlungsfluid-Beutel (18) zusammenzufalten und durch den Auslaßschlauch (20) kontrollierbar Behandlungsfluid aus diesem auszustoßen, worin die Mittel (70 oder 70a) zur elektronischen Steuerung des Drucks der Blase (68) außerdem koordinierende Aktivatormittel aufweisen, so daß durch das Einsetzen eines Behandlungsfluid-Beutels (18), der ein Aktivatormittel enthält, in das Behältnis für den Beutel (18) eine Wechselwirkung mit den Aktivatormitteln hergestellt werden kann, um dadurch die Mittel (70 oder 70a) zur elektronischen Steuerung des Drucks der Blase (68) zur Arbeit in die Lage zu versetzen.

19. Pumpe (10 oder 40) nach Anspruch 18, worin ein faltbarer Behandlungsfluid-Beutel (18) einen Aktivator einschließt und das elektronische Steuersystem (70 oder 70a) außerdem Wechselwirkungsmittel zur Wechselwirkung mit dem Aktivator einschließt, wenn dieser an einem Behandlungsfluid-Beutel (18) vorhanden ist, der innerhalb des Behältnisses angeordnet worden ist, um das elektronische Steuersystem zur Arbeit zu befähigen, wobei das elektronische Steuersystem beim Fehlen des Aktivators außer Betrieb gesetzt ist.

20. Pumpe (10 oder 40) nach Anspruch 18, worin das elektronische Steuersystem (70 oder 70a) einen Drucksensor (80) in funktioneller Verbindung mit einer geeigneten elektronischen Steuerschaltung einschließt, die wiederum in funktioneller Verbindung mit einer Antriebsfluid-Pumpe (72) steht, die funktionell mit der Blase (68) gekoppelt ist.

21. Pumpe (10 oder 40) nach Anspruch 18, die außerdem Mittel (38) zum Entlüften der Blase (68) einschließt.

## Revendications

1. Pompe (10 ou 40) pour l'administration par infusion d'un fluide de traitement intravasculaire à un patient, ladite pompe d'infusion (10 ou 40) comprenant:
un sachet de fluide de traitement pliable (18);
une vessie gonflable (68) juxtaposée audit sachet de fluide de traitement (18);
un moyen (38) pour purger ladite vessie (68);
un moyen (12, 14 ou 42, 44) pour faire heurter ladite vessie (68) contre ledit sachet de fluide de traitement (18) lors du gonflement de ladite vessie (68);
un tube de sortie (20), en communication de fluide avec ledit sachet de fluide de traitement (18) et ledit patient; et
un moyen (70 ou 70a) pour assurer la commande de la pression de ladite vessie (68) pour plier ledit sachet de fluide de traitement (18) et pour assurer une expulsion commandée du fluide de traitement;
caractérisée en ce que ledit moyen de commande (70 ou 70a) sert à assurer la commande électronique de la pression de ladite vessie (68) et comprend en outre un moyen d'activation de coordination, de sorte que le positionnement d'un sachet de fluide de traitement (18) contenant un moyen d'activation dans le moyen de confinement dudit sachet (18) permet la coopération dudit moyen d'activation, permettant ainsi le fonctionnement du moyen (70 ou 70a) destiné à assurer la commande électronique de la pression de ladite vessie (68).

2. Pompe (10 ou 40) selon la revendication 1, dans laquelle ledit tube de sortie (20) a une surface de section transversale prédéterminée et essentiellement fixe.

3. Pompe (10 ou 40) selon la revendication 1, comprenant en outre un moyen d'obturation (104) destiné à bloquer sélectivement l'écoulement du fluide de traitement à travers ledit tube de sortie (20).

4. Pompe (10 ou 40) selon la revendication 1, dans laquelle ledit moyen (70 ou 70a) destiné à assurer la commande électronique de la pression de ladite vessie (68) comprend un capteur de pression (80), communicant en service avec un circuit de commande approprié, communiquant à son tour en service avec une pompe du fluide d'entraînement (72).

5. Pompe (10 ou 40) selon la revendication 4, comprenant en outre une source d'énergie interne (92) pour ledit moyen (70 ou 70a) destiné à assurer la commande électronique de la pression de ladite vessie (68).

6. Pompe (10 ou 40) selon la revendication 1, dans laquelle ledit moyen (12, 14 ou 42, 44), destiné à entraîner le heurt comprend des premier et deuxième éléments de confinement opposés confinant ladite vessie juxtaposée (68) et ledit sachet du fluide (18).

7. Pompe (10 ou 40) selon la revendication 6, dans laquelle ledit premier élément de confinement (42) comporte une surface plane rigide, ledit deuxième élément de confinement (44) étant constitué par une feuille flexible s'engageant dans ledit premier élément de confinement.

8. Pompe (10 ou 40) selon la revendication 6, dans laquelle ledit premier élément de confinement (12) est constitué par une base rigide, ledit deuxième élément de confinement étant constituée par une plaque de couverture rigide (14) pouvant s'engager de façon amovible dans ladite base pour renfermer ledit sachet (18) et ladite vessie (68).

9. Pompe (10 ou 40) selon la revendication 1, comprenant en outre un moyen pour surveiller la quantité de fluide administrée dans le temps.

10. Sachet de fluide de traitement pliable (18) comprenant:
un set d'extension qui y est fixé;
un moyen d'activation capable de coopérer avec le moyen (70 ou 70a) destiné à assurer la commande électronique de la pression de la vessie (68) d'une pompe (10 ou 40) selon la revendication 1, ledit moyen (70 ou 70a) destiné à assurer la commande électronique de la pression de ladite vessie (68) étant bloqué en cas d'absence dudit moyen d'activation; et
un orifice pour introduire le fluide dans ledit sachet de traitement (18).

11. Sachet de traitement (18) selon la revendication 10, dans lequel ledit set d'extension comprend un moyen d'étranglement de l'écoulement incorporé (25 ou 58); un raccord (24) pour fixer une aiguille ou un site de cathéter d'un patient; une pince pour arrêter l'écoulement du fluide de traitement avant, pendant ou après l'administration du fluide de traitement au patient; et, optionnellement, un filtre d'élimination des particules dans la ligne du tube et un filtre d'évacuation de l'air monté sur le sachet du fluide de traitement (18) ou dans la ligne du tube, ainsi qu'un clapet de retenue.

12. Sachet de traitement (18) selon la revendication 11, dans lequel ledit moyen d'étranglement de l'écoulement incorporé est un moyen d'étranglement de l'écoulement fixe ou un moyen d'étranglement de l'écoulement variable à débits préréglés.

13. Sachet de traitement (18) selon la revendication 10, dans lequel ledit moyen d'activation coopère de façon électronique, mécanique, pneumatique, magnétique ou optique avec le mécanisme de commande de ladite pompe (10 ou 40).

14. Sachet de fluide de traitement (18) selon la revendication 10, dans lequel ledit sachet (18) comprend des chambres multiples (18' ou 18'') capables de retenir un fluide de traitement.

15. Sachet de fluide de traitement (18) selon la revendication 10, dans lequel ledit sachet (18) a une forme facilitant l'expulsion d'air à travers le tube d'entrée (25) ou de sortie (20).

16. Sachet de fluide de traitement (18) selon la revendication 14, dans lequel les chambres multiples (18') du sachet (18) sont agencées dans un agencement de récipient de fluide empilé.

17. Sachet de fluide de traitement (18) selon la revendication 14, dans lequel les chambres multiples (18'') du sachet (18) sont agencées dans une orientation parallèle.

18. Pompe (10 ou 40) pour administrer par infusion un fluide de traitement intravasculaire à un patient, ladite pompe d'infusion (10 ou 40) comprenant:
(1) un récipient de confinement pour recevoir un sachet de fluide de traitement amovible, pliable (18) comportant un tube de sortie (20) pour établir une communication de fluide entre ledit sachet de fluide de traitement (18) et ledit patient; et
(2) une vessie gonflable (68) juxtaposée audit sachet du fluide de traitement (18) lorsque ledit sachet de fluide de traitement (18) est positionné dans ledit récipient, de sorte que ladite vessie (68) applique une pression sur ledit sachet de fluide de traitement (18) lors du gonflement de ladite vessie (68);
caractérisée par:
(3) un système de commande électronique (70 ou 70a), destiné à assurer la commande électronique de la pression de ladite vessie (68) de sorte à plier ledit sachet de fluide de traitement (18) et à assurer l'expulsion commandée du fluide de traitement à travers ledit tube de sortie (20), ledit moyen (70 ou 70a) étant destiné à assurer la commande électronique de la pression de ladite vessie (68), et comprenant en outre un moyen d'activation de coordination, de sorte que le positionnement d'un sachet de fluide de traitement (18) contenant un moyen d'activation dans le moyen de confinement dudit sachet (18) permet la coopération dudit moyen d'activation, permettant ainsi le fonctionnement dudit moyen (70 ou 70a) destiné à assurer la commande électronique de la pression de ladite vessie (68).

19. Pompe (10 ou 40) selon la revendication 18, dans laquelle un sachet de fluide de traitement compatible (18) englobe un moyen d'activation, ledit système de commande électronique (70 ou 70a) comprenant en outre un moyen de coopération destiné à coopérer avec ledit moyen d'activation lors de son agencement sur un sachet de fluide de traitement (18) positionné dans ledit récipient pour permettre le fonctionnement dudit système de commande électronique, ledit système de commande électronique étant bloqué en cas d'absence dudit moyen d'activation.

20. Pompe (10 ou 40) selon la revendication 18, dans laquelle ledit système de commande électronique (70 ou 70a) englobe un capteur de pression (80), communiquant en service avec un circuit de commande électronique approprié, communiquant à son tour en service avec une pompe du fluide d'entraînement (72) couplée en service à ladite vessie (68).

21. Pompe (10 ou 40) selon la revendication 18, comprenant en outre un moyen (38) pour purger ladite vessie (68).
